# EUROPÄISCHE PATENTSCHRIFT

(11) **EP 1 525 015 B1**
(45) Veröffentlichungstag und Bekanntmachung des Hinweises auf die Patenterteilung: **10.10.2007**
(21) Anmeldenummer: 03732168.4
(22) Anmeldetag: 08.07.2003
(51) Int. Cl.: A61M 5/315

(54) **VERABREICHUNGSGERÄT MIT PRIMINGFUNKTION**
ADMINISTRATION DEVICE COMPRISING A PRIMING FUNCTION
APPAREIL D'ADMINISTRATION A FONCTION D'AMORÇAGE

(30) Priorität: 17.07.2002 DE 10232412
(43) Veröffentlichungstag der Anmeldung: 27.04.2005
(73) Patentinhaber: TecPharma Licensing AG, 3401 Burgdorf (CH)
(72) Erfinder: GRAF, Roney, CH-3400 Burgdorf (CH); KIRCHHOFER, Fritz, CH-3454 Sumiswald (CH)
(86) Internationale Anmeldenummer: PCT/CH2003/000457
(87) Internationale Veröffentlichungsnummer: WO 2004/007000

(56) Entgegenhaltungen:
- EP-A- 0 937 477
- WO-A-02/30495
- US-A- 5 643 214
- US-B1- 6 228 067

## Beschreibung

Die Erfindung betrifft ein Verabreichungsgerät für die Verabreichung eines fluiden Produkts für vorzugsweise medizinische einschließlich tiermedizinische, therapeutische, diagnostische, pharmazeutische und/oder kosmetische Anwendungen. Injektionsgeräte, insbesondere Injektionspens, und auch Inhalationsgeräte sind besonders bevorzugte Beispiele von Verabreichungsgeräten.

In den genannten bevorzugten Anwendungen ist es meistens wichtig, dass eine ganz bestimmte Produktdosis, d.h. eine ganz bestimmte Produktmenge, verabreicht wird. Eine Quelle der Unsicherheit ist diesbezüglich der Umstand, dass ein Teil des Geräts, der das Produkt führt, nicht gänzlich mit dem Produkt gefüllt ist, sondern Luft enthält. Ohne Entlüftung würde die Luft, oder allgemeiner ein Gas, zusammen mit dem Produkt verabreicht werden. Bei beispielsweise subkutaner Verabreichung würde dies zwar nicht zu gesundheitlichen Komplikationen führen, aber immerhin würde nicht die korrekte Produktdosis verabreicht werden, sondern eine um den Luftgehalt verminderte Dosis. Bei anderen Arten der Verabreichung kann es jedoch zusätzlich auch zu gesundheitlichen Beeinträchtigungen kommen, wenn Luft zusammen mit dem Produkt verabreicht wird. Luft kann in dem produktführenden Teil des Verabreichungsgeräts nicht nur dann vorhanden sein, wenn erstmals aus einem Reservoir verabreicht wird, sondern auch noch nach einer ersten Verabreichung wieder oder überhaupt erstmals eintreten. So kann ein flüssiges Produkt in einer Injektionskanüle beispielsweise von der Kanülenspitze her verdunsten und die Kanüle eintrocknen. Falls ein Nachfließen des Produkts von der Seite des Reservoirs her aufgrund der herrschenden Druckverhältnisse nicht oder nicht ausreichend rasch möglich ist, bildet sich in der Injektionskanüle von der Spitze her ein Luftvolumen. Wird später ohne vorherige Entlüftung erneut Produkt verabreicht, so ist die verabreichte Produktdosis diesem Luftvolumen entsprechend vermindert.

Verabreichungsgeräte, wie die Erfindung sie beispielsweise auch betrifft, sind aus der WO 97/36625, der DE 199 00 792 C1 und der US 6,228,067 B1 bekannt. Die dort beschriebenen Verabreichungsgeräte sind Injektionspens, die eine Auswahl der per Injektion zu verabreichenden Produktdosis erlauben. Um die bekannten Geräte zu primen, drückt der Verwender "nach Gefühl" Produkt durch die Injektionsnadel des jeweiligen Geräts, bis er durch Sichtkontrolle den Austritt von Produkt an der Nadelspitze feststellt. Solch ein Primen, wie das Entlüften im Allgemeinen bezeichnet wird, "auf Sicht" und "nach Gefühl" kann insbesondere darin resultieren, dass unnötig viel Produkt zum Zwecke des Primens ausgeschüttet und daher für die Verabreichung verloren geht. Für Menschen mit beeinträchtigter Sehkraft besteht darüber hinaus ein erhöhtes Risiko, dass der Primingvorgang wegen der erforderlichen Sichtkontrolle nicht korrekt durchgeführt wird, was in einem noch höheren, unnötigen Produktverbrauch oder in einer nicht vollständigen Entlüftung resultieren kann.

Aus der EP 0 937 477 ist ein Verabreichungsgerät bekannt, bei der die Primingfunktion durch Rotation einer Hülse erzielt wird.

Bei anderen Verabreichungsgeräten, beispielsweise bei Inhalationsgeräten, kann je nach Bauart auch der Fall eintreten, dass Luft in dem produktführenden Geräteteil überhaupt nicht auf Sicht überwacht werden kann und es daher von Hause aus noch schwieriger ist, die Verabreichung einer bestimmten Produktdosis zu überwachen.

Es ist eine Aufgabe der Erfindung, die Sicherheit dafür zu erhöhen, dass eine bestimmte, dem Verwender bekannte Produktdosis auch tatsächlich verabreicht wird.

Ein Verabreichungsgerät für die Verabreichung eines fluiden Produkts, wie die Erfindung es betrifft, umfasst ein Gehäuse mit einem Reservoir für das Produkt, eine Abtriebsvorrichtung, die auf das Produkt wirkt, um das Produkt auszuschütten, und eine Antriebsvorrichtung, die auf die Abtriebsvorrichtung wirkt. Die Antriebsvorrichtung ist aus einer Ausschüttposition in eine Antriebsrichtung bis gegen einen Ausschüttanschlag bewegbar. Diese Ausschüttbewegung ist vorzugsweise eine lineare Translationsbewegung, kann grundsätzlich jedoch auch entlang eines Kurvenbogens ausgeführt werden und kann auch eine Rotationsbewegung sein oder eine Rotationsbewegung umfassen. Die volle Bewegung der Antriebsvorrichtung aus der Ausschüttposition bis gegen den Ausschüttanschlag wird im Folgenden auch als Ausschütthub bezeichnet. Die Antriebsvorrichtung ist mit der Abtriebsvorrichtung so gekoppelt, dass durch die Bewegung der Antriebsvorrichtung in Richtung auf den Ausschüttanschlag die Abtriebsvorrichtung betätigt und dadurch Produkt aus dem Reservoir und durch ein sich an das Reservoir vorzugsweise anschließendes, produktführendes System ausgeschüttet wird. Das produktführende System reicht von dem Auslass des Reservoirs bis zu einer Austrittsstelle für das Produkt. Im Falle eines Injektionsgeräts mit einer Injektionskanüle, beispielsweise in Form einer hohlen Injektionsnadel, bildet im Allgemeinen die Injektionskanüle das gesamte produktführende System stromabwärts von dem Reservoir.

Das Reservoir kann von dem Gehäuse selbst gebildet werden. Bevorzugter bildet jedoch ein Produktbehältnis das Reservoir, das von dem Gehäuse, vorzugsweise in dem Gehäuse, aufgenommen ist. Insbesondere kann das Produktbehältnis eine mit dem Produkt vorabgefüllte Ampulle sein.

Nach der Erfindung ist ein Priminganschlag für die Antriebsvorrichtung vorgesehen, der einen der Entlüftung dienenden Priminghub der Antriebsvorrichtung in Antriebsrichtung begrenzt, so dass der Priminghub in Antriebsrichtung kürzer als ein maximaler Ausschütthub ist. Die Antriebsvorrichtung kann nicht nur die Ausschüttposition, sondern auch eine Primingposition einnehmen, aus der sie in die Antriebsrichtung bis gegen den Priminganschlag und quer zu der Antriebsrichtung bis in die Ausschüttposition bewegbar ist. Die Bewegung aus der Primingposition in die Ausschüttposition kann in einem rechten Winkel zur der Antriebsrichtung erfolgen oder auch in einem anderen Winkel; insoweit soll nur ausgesagt sein, dass diese Bewegung eine Querkomponente zu der Antriebsrichtung hat. Eine exakt rechtwinklig zu der Antriebsrichtung verlaufende Bewegung stellt allerdings ein bevorzugtes Ausführungsbeispiel dar. Indem die Antriebsvorrichtung nicht nur den der Produktverabreichung dienenden Ausschütthub, sondern zusätzlich den Priminghub mit definierter Länge ausführen kann, ist es möglich, das fluidführende System ohne Sichtkontrolle sicher zu entlüften. Aufgrund der definierten Länge des Priminghubs weiß der Verwender außerdem, welche Produktmenge er durch den Primingvorgang höchstens "verloren" hat. Dies kommt insbesondere Menschen zugute, die in ihrer Sehkraft beeinträchtigt sind.

Der Priminghub findet vorzugsweise zwischen zwei in Antriebsrichtung gesehen fixen Anschlagpositionen statt, von denen die eine die Primingposition ist und die andere durch den Priminganschlag definiert wird. Allerdings soll nicht ausgeschlossen sein, dass der Priminganschlag oder ein die Primingposition definierender Anschlag verstellt werden können, um die Länge des Priminghubs speziellen Wünschen in gewissen Grenzen anpassen zu können. Der Priminghub ist einerseits deutlich kürzer als der Ausschütthub, aber andererseits ausreichend lang, um unter allen Betriebsbedingungen die Entlüftung der produktführenden Teile des Verabreichungsgeräts von dem Reservoir bis zu einer stromabwärts von dem Reservoir gelegenen nächsten Austrittsstelle sicher zu gewährleisten. Der maximale Ausschütthub eines Geräts, das eine Dosisauswahl zulässt, entspricht der maximal auswählbaren Dosis. Bei Geräten, bei denen die Möglichkeit einer Dosisauswahl nicht vorgesehen ist, soll unter dem maximalen Ausschütthub ebenfalls die Länge des Hubs bezeichnet sein, mit dem die mit dem betreffenden Gerät maximal in einem Hub ausschüttbare Produktdosis ausgeschüttet wird. In diese Kategorie fallen nicht nur Geräte, bei denen mit einem einzigen Hub das gesamte Reservoir entleert wird, sondern auch Geräte, deren Reservoir durch mehrere Ausschütthübe nicht veränderbarer Länge entleert wird. In diesem Fall ist jeder der Ausschütthübe ein maximaler Ausschütthub.

Der Priminghub ist vorzugsweise wenigstens zehnmal, bevorzugter wenigstens 20 mal, kürzer als der maximale Ausschütthub. Falls eine Dosiseinheit eine kleinste verabreichbare Produktdosis darstellt und die Produktdosis nur im ganzzahligen Vielfachen der Dosiseinheit verabreicht werden kann, entspricht der minimale Priminghub einer Dosiseinheit. Bevorzugter entspricht er einigen wenigen Dosiseinheiten, beispielsweise zwei oder drei Dosiseinheiten.

In bevorzugter Ausführung ist die Antriebsvorrichtung nicht nur aus der Primingposition in die Ausschüttposition, sondern auch aus der Ausschüttposition in die Primingposition bewegbar. Besonders bevorzugt ist die Antriebsvorrichtung wahlweise aus der einen in die andere Position bewegbar. Bei einem Wegwerfgerät, dessen Reservoir mit einem einzigen Ausschütthub entleert wird, genügt es jedoch, wenn die Antriebsvorrichtung nur ein einziges Mal aus der Primingposition in die Ausschüttposition, und von dort nicht wieder zurück in die Primingposition, bewegt werden kann. Vorzugsweise kann die Antriebsvorrichtung aus der Primingposition unmittelbar in die Ausschüttposition bewegt werden, d.h. ohne Hubbewegung in die Antriebsrichtung. Grundsätzlich denkbar wäre es jedoch auch, dass aus der Primingposition in einem ersten Schritt der Priminghub ausgeführt und erst in der dann erreichten Anschlagposition durch eine Querbewegung die Antriebsvorrichtung in die Ausschüttposition gebracht wird. Die Überführmöglichkeit aus der Primingposition in die Ausschüttposition ohne zwischengeschaltete Hubbewegung in die Antriebsrichtung ist jedoch insbesondere dann von Vorteil, wenn die Antriebsvorrichtung im Rahmen einer Auswahlbewegung zwischen der Primingposition und der Ausschüttposition hin und her bewegt werden kann. In Bezug auf die Antriebsrichtung nimmt die Antriebsvorrichtung in ihrer Ausschüttposition und in ihrer Primingposition vorzugsweise die gleiche Höhe ein, so dass eine Auswahlbewegung vorteilhafterweise eine einfache Bewegung in einem rechten Winkel zu der Antriebsrichtung sein kann.

In bevorzugter Ausführung erlaubt das Verabreichungsgerät die Auswahl der zu verabreichenden Produktdosis durch den Verwender. Es umfasst deshalb ein Dosierglied, das einen Dosieranschlag bildet und mit dem Gehäuse so gekoppelt ist, dass es relativ zu dem Gehäuse eine Dosierbewegung ausführen kann. Durch die Dosierbewegung wird der Dosieranschlag relativ zu der Antriebsvorrichtung verstellt. Der Dosieranschlag liegt dem Ausschüttanschlag entgegen der Antriebsvorrichtung, vorzugsweise axial, gegenüber. Der in Antriebsrichtung gemessene Abstand zwischen dem Dosieranschlag und dem Ausschüttanschlag entspricht dem Ausschütthub der Antriebsvorrichtung.

Für die Ausführung der Dosierbewegung ist das Dosierglied vorzugsweise nur mit dem Gehäuse gekoppelt. Die Kopplung kann insbesondere ein Drehgelenk umfassen oder allein durch ein Drehgelenk gebildet werden. So kann das Dosierglied beispielsweise um eine feste Rotationsachse nur drehbar mit dem Gehäuse verbunden sein und einen treppenförmigen oder kontinuierlich spiraligen Dosieranschlag aufweisen, wie sie in der WO 97/36625 und der DE 199 00 792 C1 beschrieben werden. Bevorzugter wird das Drehgelenk durch einen unmittelbaren Gewindeeingriff zwischen dem Gehäuse und dem Dosierglied gebildet, ist also ein Schraubgelenk. Für die Ausführung der Dosierbewegung kann das Dosierglied alternativ jedoch auch mit der Antriebsvorrichtung einerseits und dem Gehäuse andererseits gekoppelt sein, vorzugsweise jeweils in einem unmittelbaren Eingriff stehen. Grundsätzlich ist auch denkbar, dass das Dosierglied für die Dosierbewegung nur mit der Antriebsvorrichtung und nicht mit dem Gehäuse gekoppelt ist, sondern von dem Gehäuse lediglich so gelagert wird, dass es seine Dosierbewegung ausführen kann.

In einer bevorzugten Ausführungsform ist der Dosieranschlag derart gebildet, dass die Antriebsvorrichtung gegen die Antriebsrichtung, d.h. von dem Ausschüttanschlag weg, bis gegen den Dosieranschlag und in die Antriebsrichtung bis gegen den Ausschüttanschlag bewegt werden kann. Die Dosierbewegung des Dosierglieds kann insbesondere eine reine axiale Translationsbewegung oder eine reine Rotationsbewegung mit der Axialen als Rotationsachse sein. Bevorzugt ist die Dosierbewegung des Dosierglieds eine axiale Translationsbewegung in Kombination mit einer Rotationsbewegung um die Axiale, wie sie beispielsweise durch das genannte Schraubgelenk bewirkt wird.

Besonders vorteilhaft ist es, wenn die Dosierbewegung des Dosierglieds eine axiale Translationsbewegung ist oder eine solche Bewegung umfasst, weil dadurch ermöglicht wird, dass der Dosieranschlag in axialer Richtung eine einzige Höhe einnimmt.

Der Priminganschlag wird vorzugsweise von dem Dosierglied gebildet. Alternativ kann das Gehäuse den Priminganschlag bilden oder auch ein zwischengeschalteter anderer Körper einer Primingmechanik beispielsweise wenn das Verabreichungsgerät ein Dosierglied gar nicht umfasst.

In der bevorzugten Ausführung, in der die Primingfunktion durch einen unmittelbaren Eingriff zwischen der Antriebsvorrichtung und dem Dosierglied erfüllt wird, umfasst solch eine Primingmechanik nur das Dosierglied und die Antriebsvorrichtung. In der alternativen Ausführung, in der die Primingfunktion durch einen Eingriff zwischen der Antriebsvorrichtung und dem Gehäuse erfüllt wird, bilden allein das Gehäuse und die Antriebsvorrichtung die Primingmechanik. Grundsätzlich ist es jedoch auch denkbar, dass die Primingmechanik einen weiteren Körper umfasst, der einerseits mit entweder dem Dosierglied oder dem Gehäuse und andererseits mit der Antriebsvorrichtung in einem Eingriff steht, um die Primingfunktion zu erfüllen Schließlich sei auch darauf hingewiesen, dass die Einbindung mehrerer weiterer Körper in solch eine Primingmechanik grundsätzlich möglich ist. Eine einfache Kopplung durch einen unmittelbaren Eingriff der Antriebsvorrichtung mit entweder dem Dosierglied oder dem Gehäuse wird jedoch bevorzugt.

Zur Erfüllung der Primingfunktion ist eine Kopplung der Antriebsvorrichtung mit dem Gehäuse anstatt mit einem Dosierglied beispielsweise dann vorteilhaft, wenn das Dosierglied einen treppenförmigen Dosieranschlag oder einen spiraligen, kontinuierlichen Dosieranschlag aufweist, wie dies aus der WO 97/36625 und der DE 199 00 792 C1 bekannt ist. Die Bewegung der Antriebsvorrichtung aus der Primingposition in die Ausschüttposition kann im Falle solch einer Ausbildung des Dosierglieds beispielsweise zuerst den Priminghub, dann eine Bewegung entlang des Ausschüttanschlags und schließlich die Bewegung gegen den Dosieranschlag umfassen. Der Ausschüttanschlag würde gleichzeitig den Priminganschlag bilden. Solch eine Ausführung der Primingmechanik ist auch in Verbindung mit dem bevorzugt axial bewegbaren Dosierglied realisierbar. In dieser Ausführung ist die Bildung der Primingmechanik durch ein Eingriff von Antriebsvorrichtung und Dosierglied jedoch nicht zuletzt deshalb von Vorteil, weil der Priminghub aus einer Position der Antriebsvorrichtung ausgeführt werden kann, in der die Antriebsvorrichtung auf Anschlag gegen den Dosieranschlag liegt.

Die Bewegungsmöglichkeiten der Antriebsvorrichtung werden vorzugsweise durch eine Führungskulisse vorgegeben, die eine Führungskurve und ein in die Führungskurve eingreifende Eingriffsglied umfasst oder vorzugsweise nur aus diesen beiden Gelenkelementen besteht. Die Führungskurve wird in bevorzugter Ausführung von zwei je in Antriebsrichtung erstreckten, voneinander getrennten Führungsabschnitten gebildet, in die das Eingriffsglied wahlweise eingreift. Ein längerer der Führungsabschnitte definiert den Ausschütthub. Ein kürzer der Führungsabschnitte definiert den Priminghub. Um das Eingriffsglied von dem einen der Führungsabschnitte in den anderen überführen zu können, weist die Führungskurve ferner einen Verbindungsabschnitt auf. Falls die Führungskurve und das Eingriffsglied an einander zugewandten Mantelflächen von beispielsweise dem Dosierglied und der Antriebsvorrichtung gebildet sind, kann der Verbindungsabschnitt von einer Ausnehmung an derjenigen der Mantelflächen gebildet sein, welche die Führungskurve bildet. Der Verbindungsabschnitt kann in solch einer Ausführungsform allerdings auch ganz einfach von einem freien Stirnende desjenigen Körpers gebildet werden, der die Führungskurve bildet.

Die Abtriebsvorrichtung ist in bevorzugten Ausführungen ein Kolben, der in dem Reservoir in eine Vorschubrichtung axial auf einen Auslass des Reservoirs zu bewegbar ist, um das Produkt aus dem Reservoir zu verdrängen. So sind Ampullen, die von den Hierstellern vorabgefüllt bereitgestellt werden, an ihrem hinteren Ende üblicherweise bereits mittels solch eines Kolbens verschlossen. Die Ausschüttbewegung der Antriebsvorrichtung ist bei Verwendung eines Kolbens bevorzugt eine axiale Linearbewegung.

Wird die Produktförderung durch einen Kolben bewirkt, so umfasst die Abtriebsvorrichtung ferner eine Kolbenstange, die unmittelbar auf den Kolben wirkt, um ihn in die Vorschubrichtung zu bewegen. Solch eine Kolbenstange kann einteilig mit dem Kolben gebildet oder separat von dem Kolben gefertigt und formschlüssig, kraftschlüssig und/oder reibschlüssig axial steif mit dem Kolben verbunden sein. Besonders bevorzugt sind der Kolben und die Kolbenstange jedoch separate Teile, und es drückt die Kolbenstange in Vorschubrichtung gegen eine Kolbenrückseite.

In bevorzugten Ausführungen wird die Kolbenstange von dem Gehäuse so gelagert, dass sie zwar in die Vorschubrichtung, aber nicht gegen die Vorschubrichtung bewegt werden kann. Eine Bewegung gegen die Vorschubrichtung ist in diesem Fall allenfalls im Zuge eines Reservoirwechsels durch entsprechende Montagehandgriffe möglich. In einfachen und besonders bevorzugten Ausführungen ist eine Rückführung der Kolbenstange jedoch nicht möglich. Das Verabreichungsgerät wird vielmehr gänzlich durch ein neues ersetzt. Denkbar wäre auch die Aufspaltung des Geräts in ein als Verbrauchsmodul konzipiertes Reservoirmodul und ein Dosier- und Antriebsmodul, das wiederholt mit neuen Reservoirmodulen verwendbar ist. Dieses Konzept wird bei Injektionsgeräten bereits mit Erfolg in Form von sogenannten semidisposable Injektionspens verfolgt und ist auch für das Verabreichungsgerät der Erfindung mit Vorteil einsetzbar.

Das fluide Produkt ist bevorzugt eine Flüssigkeit für medizinische, therapeutische, diagnostische, pharmazeutische oder kosmetische Anwendungen oder kombiniert für mehrere dieser Anwendungen. Das Produkt kann beispielsweise Insulin, ein Wachstumshormon, Cortison, Collagen oder auch eine Flüssignahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Eine Verwendung nur oder auch im stationären und ambulanten Bereich durch geschultes Personal soll jedoch nicht ausgeschlossen sein.

Die Produktverabreichung kann im Falle eines Injektionsgeräts mittels Injektionskanüle oder beispielsweise einer Düse für nadellose Injektionen erfolgen. Die Injektion kann insbesondere subkutan oder venös vorgenommen werden, oder auch intramuskulär. Das Verabreichungsgerät kann auch ein Inhalationsgerät sein, bei dem die ausgewählte Produktdosis oder eine im Reservoir noch vorhandene Restdosis aus dem Reservoir beispielsweise in eine Kammer des Inhalationsgeräts ausgeschüttet und mittels einer Zerstäubungseinrichtung oder einer sonstigen Vemebelungseinrichtung für die Inhalation vernebelt wird. Denkbar ist ferner eine orale Einnahme des Produkts oder eine Verabreichung über die Speiseröhre, um nur einige Beispiele der Verabreichung zu nennen.

In den Unteransprüchen werden weitere bevorzugte Ausgestaltungen der Erfindung beschrieben.

Ein Ausführungsbeispiel der Erfindung wird nachfolgend anhand von Figuren beschrieben. Die an dem Ausführungsbeispiel offenbar werdenden Merkmale bilden je einzeln und in jeder Merkmalskombination die Gegenstände der Ansprüche weiter. Es zeigen:
- Figur 1: ein Injektionsgerät in einem Längsschnitt,
- Figur 2: den hinteren Teil des Injektionsgeräts,
- Figur 3: das Injektionsgerät in einem Querschnitt,
- Figur 4: ein Dosierglied des Injektionsgeräts in einem Längsschnitt,
- Figur 5: ein modifiziertes Dosierglied in einem Längsschnitt,
- Figur 6: den hinteren Teil des Injektionsgeräts in einem Minimaldosis-Zustand und "nicht geladen",
- Figur 7: den hinteren Teil des Injektionsgeräts in einem Maximaldosis-Zustand und "nicht geladen",
- Figur 8: den hinteren Teil des Injektionsgeräts in dem Maximaldosis-Zustand und "geladen",
- Figur 9: eine Ansicht auf eine Dosisanzeige in dem Gerätezustand der Figur 6,
- Figur 10: die Ansicht auf die Dosisanzeige nach einem "Ladevorgang" aus dem Zustand der Figur 9,
- Figur 11: die Ansicht auf die Dosisanzeige in dem Gerätezustand der Figur 7,
- Figur 12: die Ansicht auf die Dosisanzeige in dem Gerätezustand der Figur 8 und
- Figur 13: eine Dosisskala abgewickelt in die Blattebene.

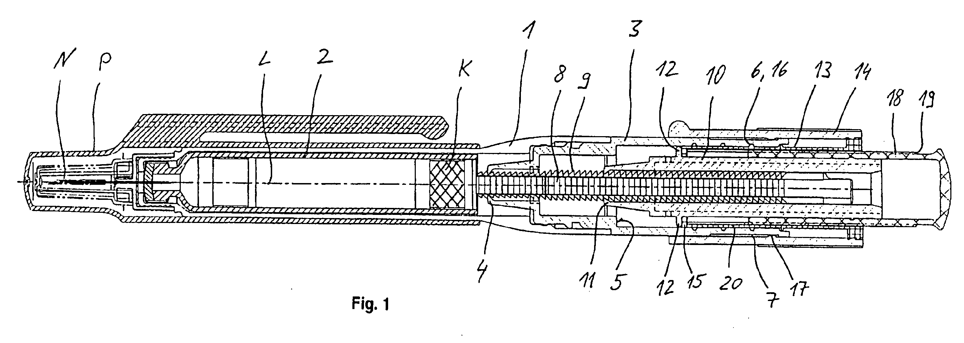

Figur 1 zeigt in einem Längsschnitt ein Injektionsgerät komplett. Es handelt sich um einen Zahnstangenpen für die Selbstverabreichung von beispielsweise Insulin oder Wachstumshormonen. Ein erster vorderer Gehäuseabschnitt 1 und ein zweiter, hinterer Gehäuseabschnitt 3 bilden eine Gehäuse des Pens. Die Gehäuseabschnitte 1 und 3 sind Hülsenkörper. Zur Bildung des Gehäuses sind sie entlang einer gemeinsamen Mittellängsachse L ineinander gesteckt und unlösbar verbunden. Die Verbindung ist ferner so, dass die Gehäuseabschnitte 1 und 3 relativ zueinander weder axial noch rotativ um die Längsachse L bewegt werden können.

Eine Ampulle 2, die in dem vorderen Gehäuseabschnitt 1 aufgenommen ist, bildet ein Reservoir für ein Produkt, das mit dem Pen im Wege einer Injektion verabreicht wird. Ein Auslass der Ampulle 2 wird von einer Membran verschlossen. Allerdings ist eine Injektionsnadel N durch die Membran gerührt und ragt mit einem hinteren Ende in die Ampulle 2 hinein. An einem von dem Auslass abgewandten, hinteren Ende der Ampulle 2 ist ein Kolben K aufgenommen. Durch eine axiale Bewegung des Kolbens K entlang der Längsachse L in eine auf den Ampullenauslass weisende Vorschubrichtung wird Produkt aus der Ampulle 2 und durch die Injektionsnadel N hindurch ausgeschüttet und dadurch verabreicht. Im dargestellten Zustand sind der vordere Gehäuseabschnitt 1 von einer äußeren Schutzkappe P und die Injektionsnadel N noch einmal separat von einer inneren Schutzkappe abgedeckt, die vor der Verabreichung selbstverständlich abgenommen werden müssen.

Der hintere Gehäuseabschnitt 3 bildet einen Mechanikhalter, indem er eine auf den Kolben K in Vorschubrichtung wirkende Kolbenstange 8 und die weiteren Komponenten des Pens lagert, die an einer Auswahl einer auszuschüttenden Produktdosis und einer Ausschüttung der ausgewählten Produktdosis beteiligt sind. Diese weiteren Komponenten sind ein Antriebsglied 10 und ein Betätigungselement 18, die zusammen eine Antriebsvorrichtung 10/18 für die Kolbenstange 8 bilden, und ferner ein Dosierglied 13/14, das im Zusammenwirken mit dem hinteren Gehäuseabschnitt 3 der Auswahl der aus der Ampulle 2 auszuschüttenden Produktdosis dient.

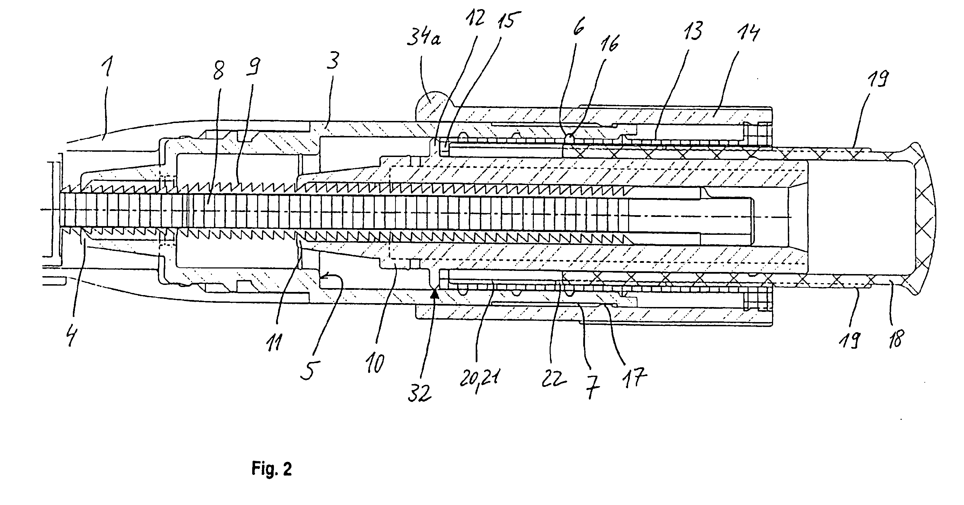

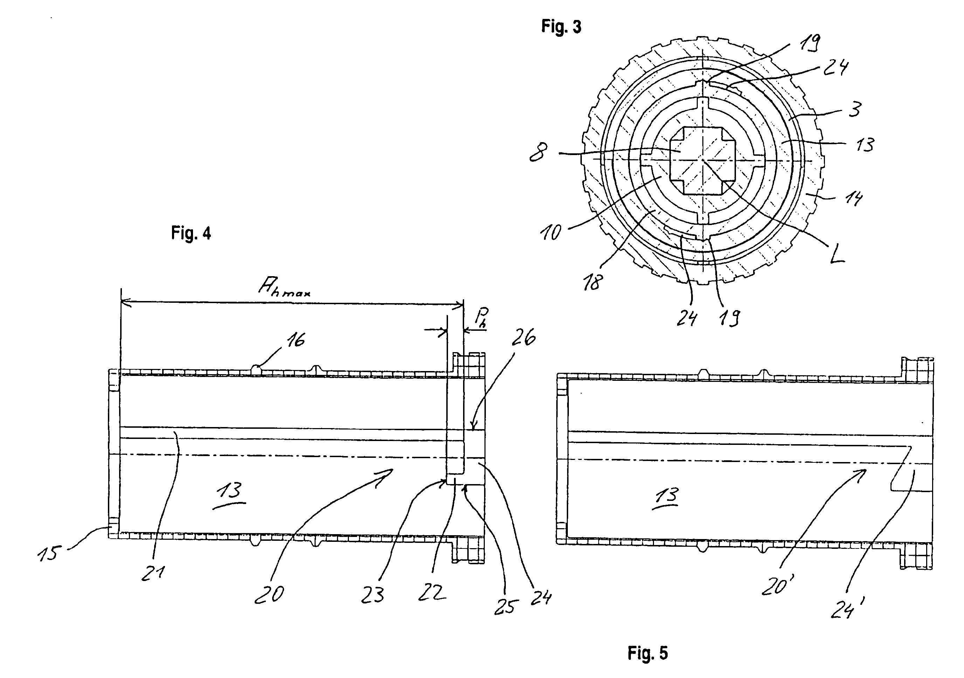

In Figur 2 sind diese Komponenten und ihre Kopplungen vergrößert dargestellt. Auch auf die Querschnittsdarstellung der Figur 3 sei stets ergänzend hingewiesen.

An dem Außenmantel der Kolbenstange 8 erstrecken sich in Längsrichtung jeweils um 90° zueinander versetzt vier aus Sägezähnen gebildete Zahnreihen 9. Die Zahnreihen 9 weisen je die gleiche Teilung auf. Jede der Zahnreihen 9 ist gegenüber jeder der anderen Zahnreihen 9 um eine viertel Zahnteilung versetzt, um die Feinheit der Dosierung in an sich bekannter Weise zu verbessern.

Der hintere Gehäuseabschnitt 3 bildet Sperrelemente 4, die in die Zahnreihen 9 eingreifen. Durch den Eingriff der Sperrelemente 4 wird die Kolbenstange 8 so blockiert, dass eine Bewegung der Kolbenstange 8 gegen die Vorschubrichtung des Kolbens K nicht möglich ist. Die Bewegung der Kolbenstange 8 in die Vorschubrichtung wird allerdings zugelassen.

Um den Vorschub der Kolbenstange 8 zu bewirken, greift hinter den Sperrelementen 4 die Antriebsvorrichtung 10/18 mit Mitnehmern 11 in die Zahnreihen 9 der Kolbenstange 8 ein. Die Antriebsvorrichtung 10/18 wird von dem hülsenförmigen Antriebsglied 10 und dem hülsenförmigen Betätigungselement 18 gebildet. Das Betätigungselement 18 ist von hinten auf das Antriebsglied 10 aufgeschoben. Die Verbindung zwischen dem Antriebsglied 10 und dem Betätigungselement 18 ist derart, dass das Betätigungselement 18 relativ zu dem Antriebsglied 10 axial nicht bewegbar, aber um die Längsachse L relativ zu dem Antriebsglied 10 drehbar ist. Die Längsachse L bildet eine Translationsachse der Antriebsvorrichtung 10/18 und der aus dem Kolben K und der Kolbenstange 8 bestehenden Abtriebsvorrichtung K, 8 und ferner eine Rotationsachse des Betätigungselements 18.

Die Antriebsvorrichtung 10/18 im Ganzen gesehen durchragt das Dosierglied 13/14. Die Mitnehmer 11 des Antriebsglieds 10 können frei, insbesondere frei von dem Dosierglied 13/14, in radialer Richtung elastisch nach außen aus dem Zahneingriff abgebogen werden, um eine Bewegung der Antriebsvorrichtung 10/18 relativ zu der Kolbenstange 8 entgegen der Vorschubrichtung zu ermöglichen. Die Mitnehmer 11 sind in einem nach vom aus dem Dosierglied 13/14 ragenden Abschnitt der Antriebsvorrichtung 10/18 gebildet; im Ausfiihrungsbeispiel bilden sie das vordere Ende der Antriebsvorrichtung 10/18. Der Eingriff der Mitnehmer 11 und die Form der Zahnreihen 9 sind derart, dass die Kolbenstange 8 durch eine Bewegung der Antriebsvorrichtung 10/18 in eine auf den Kolben K gerichtete Antriebsrichtung, die mit der Vorschubrichtung identisch ist, zwangsweise mitgenommen wird.

Im Ausführungsbeispiel sind die Zahnreihen 9 sägezahnförmig in Vorschubrichtung gepfeilt. In modifizierten Ausbildungen könnte die Kolbenstange 8 jedoch statt einer oder mehreren Zahnreihen 9 Vertiefungen oder andere Eingriffseinrichtungen für Mitnehmer einer Antriebsvorrichtung aufweisen, wenn nur sichergestellt ist, dass eine zwangsweise Mitnahme in die Vorschubrichtung erfolgt, aber eine Mitnahme gegen die Vorschubrichtung verhindert wird.

Das Dosierglied 13/14 wird von einem hülsen%rmigen inneren Dosierkörper 13 und einem hülsenförmigen äußeren Dosierkörper 14 gebildet, die als separate Teile gefertigt und unbeweglich miteinander verbunden sind. Insbesondere ist zwischen den beiden Dosierkörpern 13 und 14 weder eine axiale Relativbewegung noch eine Relativdrehung um die Längsachse L möglich. Der äußere Dosierkörper 14 umgibt den inneren Dosierkörper 13 konzentrisch. Zwischen den beiden Dosierkörpern 13 und 14 verbleibt umlaufend ein lichter Ringspalt, in den der ebenfalls hülsenförmige hintere Gehäuseabschnitt 3 hineinragt.

Der hintere Gehäuseabschnitt 3 bildet mit dem Dosierkörper 13/14 ein Drehgelenk, genauer gesagt ein Schraubgelenk, welches bewirkt, dass eine Rotationsbewegung des Dosierglieds 13/14 um die Längsachse L in Abhängigkeit von der Drehrichtung eine Translationsbewegung des Dosierglieds 13/14 in oder gegen die Vorschubrichtung, also eine axiale Translationsbewegung, relativ zu dem hinteren Gehäuseabschnitt 3 zur Folge hat. Die Längsachse L bildet eine Rotationsachse und eine Translationsache des Dosierglieds 13/14. Zur Bildung des Drehgelenks sind der hintere Gehäuseabschnitt 3 und der Dosierkörper 13/14 in einem Gewindeeingriff. Der Gewindeeingriff besteht zwischen einem Innengewinde 6, das an einer Innenmantelfläche des hinteren Gehäuseabschnitts 3 geformt ist, und einem Außengewinde 16, das an einer Außenmantelfläche des inneren Dosierkörpers 13 geformt ist. Der Gewindeeingriff könnte, obgleich weniger bevorzugt, stattdessen auch zwischen einem Außengewinde des hinteren Gehäuseabschnitts 3 und einem eingreifenden Innengewinde des äußeren Dosierkörpers 13 gebildet sein. Durch Rotation des Dosierkörpers 13/14 wählt der Verwender die zu verabreichende Produktdosis. Indem durch den Gewindeeingriff das Dosierglied 13/14 gleichzeitig auch eine axiale Translationsbewegung ausführt, wird ein Ausschütthub der Länge Ah für die Antriebsvorrichtung 10/18 eingestellt, welcher der ausgewählten Produktdosis entspricht.

Der innere Dosierkörper 13 bildet einen Dosieranschlag 15, der die Bewegung der Antriebsvorrichtung 10/18 gegen die Vorschubrichtung begrenzt. Den Dosieranschlag 15 bildet genauer gesagt an seinem vorderen Ende eine in Vorschubrichtung weisende Stirnfläche des Dosieranschlags 15. Der Dosieranschlag 15 wird von einer um die Längsachse L umlaufenden Ringschulter gebildet, die nach radial einwärts über eine Innenmantelfläche des inneren Dosierkörpers 13 auf das Antriebsglied 10 zu vorsteht. Der Dosieranschlag 15, genauer gesagt dessen vordere Anschlagfläche, läuft auf einer axial konstanten Höhe um die Längsachse L um, d.h. es wird ein gerader Dosieranschlag 15 auf einer einzigen Höhe gebildet.

Dem Dosieranschlag 15 in Vorschubrichtung gegenüber bildet der hintere Gehäuseabschnitt 3 einen Ausschüttanschlag 5, der die Bewegung der Antriebsvorrichtung 10/18 in die Vorschubrichtung begrenzt. Die Antriebsvorrichtung 10/18 kann somit zwischen dem gehäusefesten Ausschüttanschlag 5, der sogar von dem hinteren Gehäuseabschnitt 3 selbst gebildet wird, und dem translativ verstellbaren Dosieranschlag 15 in und gegen die Vorschubrichtung bewegt werden. Die Antriebsvorrichtung 10/18 bildet ihrerseits an einer Außenmantelfläche des Antriebsglieds 10 einen Gegenanschlag, nämlich den Ausschütt- und Dosieranschlag 12. Der Ausschütt- und Dosieranschlag 12 wird von einer Ringschulter mit einer dem Ausschüttanschlag 5 zugewandten vorderen Anschlagfläche und einer dem Dosieranschlag 15 zugewandten hinteren Anschlagfläche gebildet. Der Ausschütt- und Dosieranschlag 12 steht von einer Außenmantelfläche des Antriebsglieds 10 radial nach außen auf eine zugewandte Innenmantelfläche des hinteren Gehäuseabschnitts 3 zu vor.

Der hintere Gehäuseabschnitt 3 ist gänzlich oder zumindest in dem Abschnitt durchsichtig, der einen maximalen Ausschütthub der Antriebsvorrichtung 10/18 überdeckt. Die Durchsichtigkeit dient der Verifizierung der axialen Position der Antriebsvorrichtung 10/18, insbesondere der Position des mit der Kolbenstange 8 unmittelbar in Eingriff stehenden und über den Eingriff in Vorschubrichtung steif verbundenen Antriebsglieds 10.

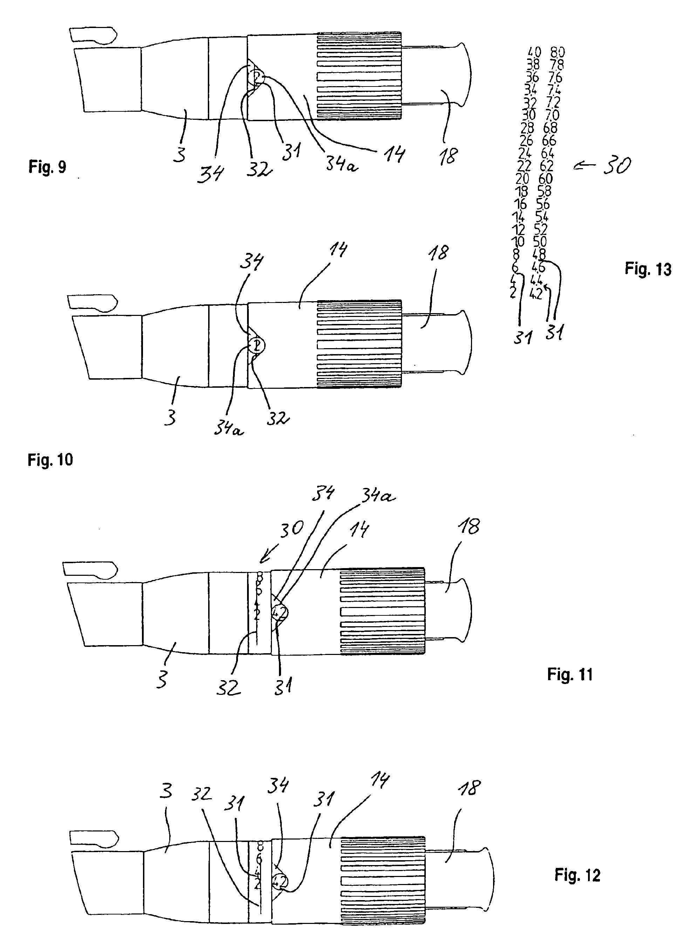

Zum Zwecke einer besonders genauen optischen Verifikation ist das Antriebsglied 10 mit einer dünnen Markierungslinie versehen, bevorzugt eine umlaufende Markierungslinie, die durch den hinteren Gehäuseabschnitt 3 hindurch gut sichtbar ist und einen Zeiger 32 bildet (Figuren 9 bis 11). Die Markierungslinie kann vorteilhafterweise von dem Ausschütt- und Dosieranschlag 12 selbst gebildet werden, indem der Ausschütt- und Dosieranschlag 12 radial außen eine dünne Spitze bildet. Der Ausschütt- und Dosieranschlag 12 bietet sich als Zeiger für die Verifikation bereits deshalb an, weil er radial bis fast unmittelbar an den hinteren Gehäuseabschnitt 3 ragt oder diesen sogar berührt und Gleitkontakt hat. Eine radial äußere Mantelfläche des Ausschütt- und Dosieranschlags 12 oder die genannte, radial äußere Spitze ist vorzugsweise zusätzlich mit einer auf der Oberfläche oder in einer Vertiefung an der Oberfläche angebrachten oder in dem Material gebildeten dünnen Markierungslinie versehen, die vorteilhafterweise fluoreszierend sein kann.

Wie in Verbindung mit den Figuren 9 bis 12 und insbesondere in Figur 13 erkennbar, weist der hintere Gehäuseabschnitt 3 über die Länge eines maximalen Ausschütthubs Ahmax eine Dosisskala 30 auf. Die Dosisskala 30 kann auf der Innenmantelfläche oder bevorzugter der Außenmantelfläche angebracht oder in dem durchsichtigen Mantel des hinteren Gehäuseabschnitts 3 eingelassen sein. Im Ausführungsbeispiel ist sie auf der äußeren Mantelfläche angebracht. Die Dosisskala 30 wird gebildet von Dosiszahlen, die Dosiseinheiten bezeichnen, und Dosismarken 31. Jeder der Dosiszahlen ist je eine der Dosismarken 31 zugeordnet. Die Dosismarken 31 werden von kurzen, dünnen Linien gebildet, die sich je in Umfangsrichtung des hinteren Gehäuseabschnitts 3 erstrecken. Die Dosiszahlen sind nach hinten aufsteigend in Form einer Spirale um die Rotationsachse L des Dosierglieds 13/14 über den Umfang des Gehäuseabschnitts 3 angeordnet. Entsprechend ergibt sich die spiralige Anordnung der Dosismarken 31 derart, dass jede der Dosismarken 31 eine andere axiale Höhe aufweist als jede der anderen Dosismarken 31. Die Dosismarken 31 bilden somit in feiner axialer Stufung jede in ganzen Dosiseinheiten auswählbare und ausschüttbare Produktdosis ab. Aufgrund der Durchsichtigkeit des hinteren Gehäuseabschnitts 3 kann die axiale Position der Antriebsvorrichtung 10/18 abgelesen werden, nämlich als die axiale Position, die der Zeiger 32 der Antriebsvorrichtung 10/18 relativ zu den Dosismarken 31 einnimmt. Im Ausführungsbeispiel bildet die genannte Markierungslinie, die an der radial äußeren Spitze des Ausschütt- und Dosieranschlags 12 um die Längsachse L umläuft, den Zeiger 32 der Antriebsvorrichtung 10/18.

Um die Ablesung der Dosisskala 30 zu erleichtern, insbesondere für Menschen mit geringer Sehschärfe, weist der äußere Dosierkörper 14 in einem Mantelabschnitt, der die Dosisskala 30 überdeckt, ein Sichtfenster 34 auf, das zu einer Lupe 34a weitergebildet ist. Das Sichtfenster 34 ist so groß und von solcher Form, dass genau eine der Dosiszahlen der Dosisskala 30 als die ausgewählte Dosis durch das Sichtfenster 34 identifiziert werden kann. Im Ausführungsbeispiel kann stets nur genau eine Dosiszahl durch den Sichtfenster abgelesen werden.

Damit die Dosisauswahl in diskreten Schritten, die den Dosiszahlen der Dosisskala 30 entsprechen, vorgenommen wird, sind das Dosierglied 13/14 und der hintere Gehäuseabschnitt 3 in diskreten Drehwinkelpositionen, die das Dosierglieds 13/14 relativ zu dem Gehäuseabschnitt 3 einnehmen kann, je in einem lösbaren Rasteingriff. Der Rasteingriff wird zwischen axial sich erstreckenden Nuten 7 und in die Nuten 7 eingreifenden Eingriffsgliedern 17 gebildet. Im Ausführungsbeispiel sind die axialen Nuten 7 an der äußeren Mantelfläche in den hinteren Gehäuseabschnitt 3 eingelassen. Entsprechend sind die Eingriffselemente 17 als kurze Rastnocken an der zugewandt inneren Mantelfläche des äußeren Dosierkörpers 14 geformt. Die Eingriffselemente 17 werden in den Axialnuten 7 in jeder der diskreten Drehwinkelpositionen des Dosierglieds 13/14 axial lineargeführt.

Eine hintere Endposition des Dosierglieds 13/14 wird durch Anschlag der Eingriffsglieder 17 an einer hinteren Stirnbegrenzungsfläche der Axialnuten 7 definiert. Eine vordere Endposition des Dosierglieds 13/14 wird durch Anschlag eines hinteren Verbindungsstegs, den die Dosierkörper 13 und 14 zwischen sich bilden, gegen eine hintere Stirnfläche des hinteren Gehäuseabschnitts 3 definiert. Zwischen diesen beiden extremen Dosierpositionen kann das Dosierglied 13/14 von Drehwinkelrastposition zu Drehwinkelrastposition hin und her verstellt werden, um den Dosieranschlag 15 axial zu verstellen. Der maximale Ausschütthub Ahmax der Antriebsvorrichtung 10/18 ist so groß wie der axiale Abstand, den der Dosieranschlag 15 in der hinteren Endstellung des Dosierglieds 13/14 von dem Ausschüttanschlag 5 aufweist abzüglich der axialen Stärke des Ausschütt- und Dosieranschlags 12.

Obgleich der Verwender die eingestellte Produktdosis jederzeit frei zwischen den beiden extremen Dosierpositionen verändern kann, ist für die meisten Anwendungen vorteilhaft, dass eine einmal eingestellte Dosis nicht mehr verändert werden muss. Das Injektionsgerät ist daher einerseits flexibel auf die Bedürfnisse unterschiedlicher Verwender einstellbar und andererseits in der für einen bestimmten Verwender optimalen Einstellung für eine wiederholte Verabreichung der jeweils gleichen Produktdosis verwendbar. In diesem Sinne wird durch das kombiniert rotatorisch und translatorisch relativ zu dem hinteren Gehäuseabschnitt 3 bewegbare und dadurch verstellbare Dosierglied 13/14 auch gleich ein Dosismemory erhalten.

Ein Vorteil, der durchaus nicht zu vernachlässigen ist, besteht darin, dass die Maßhaltigkeit und einfache Kontrolle der Maßhaltigkeit und die stabile Bauweise der Komponenten, die für die Dosierung und Ausschüttung maßgeblich sind, mit sehr wenigen Komponenten realisiert werden. Dies trägt dazu bei, dass das Gerät einfach aufgebaut und nicht zuletzt deshalb preiswert ist, aber dennoch präzise arbeitet und eine exakte Dosierung gewährleistet.

Vorteilhaft ist auch die ineinander geschachtelte Anordnung der Dosierkörper 13 und 14, wodurch eine Multifunktionalität des Dosierglieds 13/14 trotz einfacher Konstruktion erhalten wird. So bildet das Dosierglied 13/14 unmittelbar den Dosiereingriff mit dem Gehäuse des Injektionsgeräts, den Dosieranschlag 15 für die Antriebsvorrichtung 10/18, ein Griffteil für den Verwender und einen Teil einer doppelten Dosisanzeige, nämlich zum einen die Anzeige der ausgewählten Dosis und zum anderen die Anzeige der ausschüttbaren Dosis.

Als einen weiteren Vorteil ermöglicht das Injektionsgerät ein einfaches und sicheres Primen, d.h. ein Entlüften der produktführenden Teile zwischen dem Kolben K und der Austrittsöffnung der Injektionsnadel N.

Zur Erläuterung der Primingfunktion wird auf die Figuren 1 und 2 und insbesondere auch auf die Figuren 3, 4 und 5 hingewiesen. Um die Primingfunktion zu erfüllen, sind der innere Dosierkörper 13 und das Betätigungselement 18 in einem Eingriff. Der Eingriff besteht zwischen einer Führungskurve und einem in die Führungskurve eingreifenden Eingriffsglied. Das Eingriffsglied ist in der Führungskurve zwischen Axialanschlägen einerseits und Radialanschlägen andererseits in einer definierten Art und Weise bewegbar. Die Führungskurve zum einen und das Eingriffsglied zum anderen sind an den einander zugewandten Mantelflächen des inneren Dosierkörpers 13 und des Betätigungselements 18 gebildet.

Wie in den Figuren 3 und 4 gezeigt, ist das mit 19 bezeichnete Eingriffsglied an einer Außenmantelfläche des Betätigungselements 18 geformt, während die mit 20 bezeichnete Führungskurve in die zugewandte Innenmantelfläche des inneren Dosierkörpers 13 eingelassen ist. Die Führungskurve 20 weist einen langen Axialabschnitt 21 und einen demgegenüber kürzeren Axialabschnitt 22 und einen Verbindungsabschnitt 24 auf, der sich in Umfangsrichtung erstreckt und die beiden Axialabschnitte 21 und 22 an ihren hinteren Enden miteinander verbindet. Die beiden im Bereich des Verbindungsabschnitts 24 in Umfangsrichtung sich gegenüberliegenden Seitenwände der Axialabschnitte 21 und 22 bilden Rotationsanschläge 25 und 26 für das Eingriffsglied 19. Der lange Axialabschnitt 21 erstreckt sich in Vorschubrichtung bis zu dem Dosieranschlag 15, genauer gesagt bis zu der hinteren Stirnfläche der Ringschulter, die den Dosieranschlag 15 bildet. Der kürzere Axialabschnitt 22 verläuft parallel zu dem Axialabschnitt 21 und ist als kurze Sacknut ausgebildet. Eine vordere Stirnfläche des Axialabschnitts 22 bildet einen Priminganschlag 23. Die Axialabschnitte 21 und 22 münden an dem hinteren Stirnende des inneren Dosierkörpers 13. Der Verbindungsabschnitt 24 ist entsprechend an dem hinteren Stirnende offen. Das Eingriffsglied 19 wird von einer Axialrippe gebildet, die an dem Außenmantel des Betätigungselements 18 geformt ist. Diese Axialrippe bildet den Priminggegenanschlag an ihrer freien vorderen Stirnfläche.

Durch den Eingriff des Eingriffsglieds 19 in die derart gebildete Führungskurve 20 kann das Betätigungselement 18 relativ zu dem inneren Dosierkörper 13 die der Führungskurve 20 entsprechende Form der Bewegung ausführen, nämlich jeden ausgewählten Ausschütthub Ah bis hin zum maximalen Ausschütthub Ahmax, den demgegenüber kürzeren Priminghub Ph und die rotatorische Auswahlbewegung. Da das Betätigungselement 18 mit dem Antriebsglied 10 axial nicht bewegbar, aber um die Längsachse L verdrehbar verbunden ist, kann das Betätigungselement 18 ohne Einwirkung auf das Antriebsglied 10 zwischen den beiden Rotationsanschlägen 25 und 26 hin und her gedreht werden. Die axiale Translationsbewegung in entweder dem langen Axialabschnitt 21 oder dem kurzen Axialabschnitt 22 ist dann jedoch nur zusammen mit dem Antriebsglied 10 möglich.

Die Rotationsbewegung des Betätigungselements 18 zwischen den beiden Rotationsanschlägen 25 und 26, d.h. aus der Ausschüttposition in die Primingposition und umgekehrt, ist wegen der Anordnung des Verbindungsabschnitts 24 an den hinteren Enden der Axialabschnitte 21 und 22 nur möglich, wenn das Antriebsglied 10 mit seinem Ausschütt- und Dosieranschlag 12 auf Anschlag gegen den Dosieranschlag 15 liegt. In dieser "geladenen" Stellung kann das Eingriffsglied 19 durch Verdrehung des Betätigungselements 18 relativ zu dem inneren Dosierkörper 13 gegen den Rotationsanschlag 25 und somit in die axiale Flucht zu dem Priminganschlag 23 bewegt werden. In dieser Drehwinkelposition des Betätigungselements 18, der Primingposition, kann die Antriebsvorrichtung 10/18 durch axialen Druck auf das Betätigungselement 18 bis gegen den Priminganschlag 23 in Vorschubrichtung bewegt werden. Die axiale Länge Ph dieses Priminghubs beträgt nur wenige Dosiseinheiten, beispielsweise zwei, drei oder vier Dosiseinheiten. Entsprechend kurz ist der axiale Abstand zwischen dem Priminganschlag 23 und dem Priminggegenanschlag, den das Eingriffsglied 19 bildet.

Vorteilhaft ist es, wenn das Betätigungselement 18 mit dem inneren Dosierkörper 13 in der Flucht der Axialabschnitte 21 und 22, d.h. an den Rotationsanschlägen 25 und 26, jeweils in einem lösbaren Rasteingriff ist. Um in der Ausschüttposition und in der Primingposition je den Rasteingriff mit dem inneren Dosierkörper 13 zu erhalten, ist das Eingriffsglied 19 an seiner schmalen äußeren Mantelfläche nochmals mit einem dünnen, axialen Rastnocken versehen, der in den beiden Rotationsanschlagpositionen des Betätigungselements 18 jeweils in einer von zwei entsprechend in der Führungskurve 20 geformten, axialen Rastnuten zu liegen kommt.

Im Ausführungsbeispiel sind zwei identische Führungskurven 20 und Eingriffsglieder 19 vorgesehen, die einander diametral gegenüberliegen.

In der Variante der Figur 4 verläuft der Verbindungsabschnitt 24 auf einer einzigen axialen Höhe einfach gerade.

Figur 5 zeigt eine Führungskurve 20 mit einem Verbindungsabschnitt 24', dessen vordere Führungswand von dem Priminganschlag 23 aus schräg nach hinten in den langen Axialabschnitt 21 führt. Aufgrund des schrägen Verlaufs des Verbindungsabschnitts 24' wird bei der Rückdrehbewegung des Betätigungselements 18 von dem Rotationsanschlag 25 auf den Rotationsanschlag 26 zu eine Translationsbewegung des Betätigungselements 18 und damit zusammen des Antriebsglieds 10 gegen die Vorschubrichtung und relativ zu der Kolbenstange 8 bewirkt. Nach der Ausführung eines Primingvorgangs muss der Verwender deshalb die Antriebsvorrichtung 10/18 nicht wieder extra relativ zu der Kolbenstange 8 zurückziehen, d.h. laden. Die Aufzieh- bzw. Ladebewegung wird vielmehr durch die Rückdrehbewegung erzwungen. Ansonsten entspricht die Führungskurve 20 der Figur 5 der Führungskurve 20 der Figur 4.

Nachfolgend wird die Funktionsweise des Injektionsgeräts beschrieben.

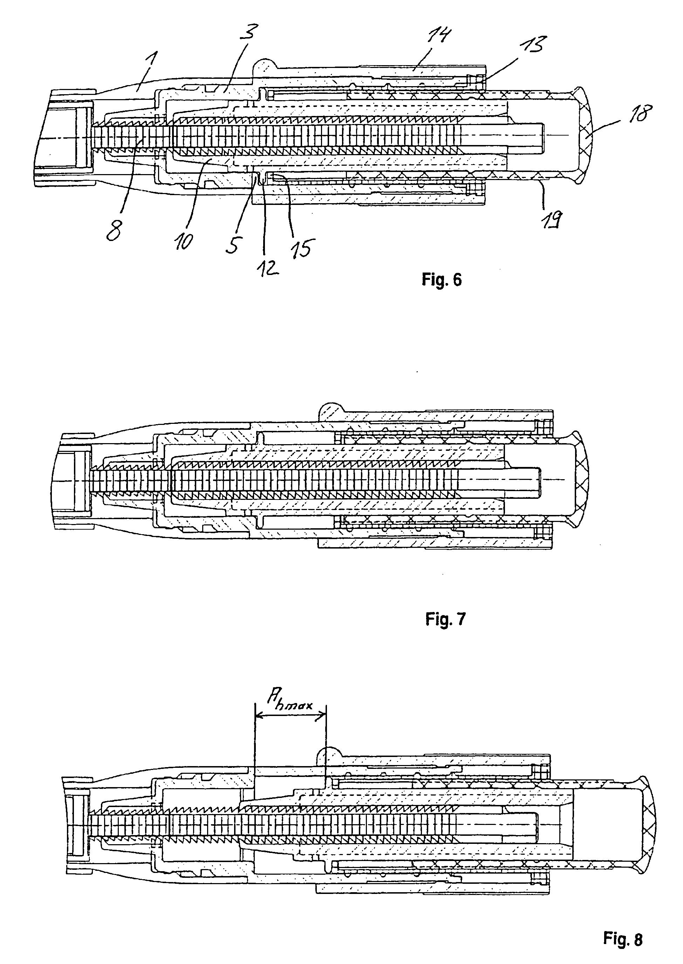

Die Figuren 6 und 9 zeigen den hinteren Teil des Injektionsgeräts in einem Längsschnitt und in einer Ansicht je in einem Zustand, aus dem heraus die Dosisauswahl vorgenommen wird. Dementsprechend liegt die Antriebsvorrichtung 10/18 auf Anschlag gegen den Ausschüttanschlag 5, und das Dosierglied 13/14 nimmt seine axial vordere Endposition ein. Zwischen dem Ausschütt- und Dosieranschlag 12 der Antriebsvorrichtung 10/18 und dem Dosieranschlag 15 des Dosierglieds 13/14 verbleibt ein lichter Abstand, der zwei Dosiseinheiten entspricht. Demgemäß kann in dem Sichtfenster 34 die Dosis von zwei Dosiseinheiten abgelesen werden. Deutlich erkennbar ist ferner auch die dieser Dosis zugeordnete Dosismarke 31. Da die Antriebsvorrichtung 10/18 mit ihrem Ausschütt- und Dosieranschlag 12 gegen den Ausschüttanschlag 5 auf Anschlag liegt, verläuft die den Zeiger 32 bildende Markierungslinie der Antriebsvorrichtung 10/18 parallel mit einem axialen Abstand zu der Dosismarke 31. Der Abstand entspricht zwei Dosiseinheiten. Diese Situation ist in Figur 9 erkennbar. Durch Zurückziehen der Antriebsvorrichtung 10/18 relativ zu der Kolbenstange 8 bis gegen den Dosieranschlag 15 wird das Injektionsgerät "geladen". Wenn diese Ladebewegung durchgeführt ist, können zwei Dosiseinheiten durch anschließendes Vorschieben der Antriebsvorrichtung 10/18 und der dabei mitgenommenen Kolbenstange 8 ausgeschüttet werden. Figur 10 zeigt diesen Zustand, in dem die Antriebsvorrichtung 10/18 mit ihrem Ausschütt- und Dosieranschlag 12 gegen den Dosieranschlag 15 des Dosierglieds 13/14 auf Anschlag liegt. Die der Dosiszahl "2" zugeordnete Dosismarke 31 und der Zeiger 32 überdecken einander exakt.

Aus dem in den Figuren 6 und 9 dargestellten Zustand heraus nimmt der Verwender seine Dosisauswahl vor. Für die Dosisauswahl muss lediglich das Dosierglied 13/14 um die Längsachse L relativ zu dem hinteren Gehäuseabschnitt 3 verdreht werden. Hierbei bilden der äußere Dosierkörper 14 ein Griffteil und der innere Dosiskörper 13 eine Dosierschraube, deren Drehbewegung durch den Gewindeeingriff unmittelbar in die axiale Verstellbewegung zum Zwecke der Dosisauswahl umgewandelt wird. Dabei erfolgt die Dosierung in diskreten Drehwinkelrastpositionen, die das Dosierglied 13/14 relativ zu dem hinteren Gehäuseabschnitt 3 sukzessive einnimmt.

Die Figuren 7 und 11 zeigen das Injektionsgerät unmittelbar nach der Dosisauswahl. Eingestellt ist eine Dosis von 42 Dosiseinheiten, wie im Sichtfenster 34 in Figur 11 abgelesen werden kann. In dem in Figur 7 dargestellten Zustand ist die Dosisauswahl abgeschlossen, das Injektionsgerät ist jedoch noch nicht "geladen". Die Antriebsvorrichtung 10/18 liegt nämlich noch auf Anschlag gegen den Ausschüttanschlag 5. Dies wird durch den Zeiger 32 auf der Dosisskala 30 angezeigt, wie in Figur 11 zu erkennen ist.

Die Figur 8 zeigt eine Maximaldosierung. Figur 12 zeigt das Injektionsgerät in einem Zustand, in dem die Dosisauswahl abgeschlossen ist, aber die Antriebsvorrichtung 10/18 nicht um den hierdurch definierten Ausschütthub der Länge Ah, sondern nur um einen Teil dieses Hubs zurückgezogen wurde. In dem in Figur 12 beispielhaft dargestellten Zustand wurde die Antriebsvorrichtung 10/18 um einen Hub zurückgezogen, der einer Produktdosis von acht Dosiseinheiten entspricht. Dies zeigt die axiale Position des Zeiger 32 auf der Dosisskala 30 in Figur 12 an.

Wie Figur 12 verdeutlicht, wird durch die Durchsichtigkeit des hinteren Gehäuseabschnitts 3, die Bildung des Zeigers 32 und die Anordnung der Dosisskala 30 eine einfache, aber zuverlässige Restmengenanzeige erhalten. Enthält die Ampulle 2 nämlich vor dem Zurückziehen der Antriebsvorrichtung 10/18 nicht mehr die volle ausgewählte Produktdosis von beispielsweise 42 Dosiseinheiten, so kann die Antriebsvorrichtung 10/18 gegen die Vorschubrichtung nur soweit zurückgezogen werden, dass die Länge ihres nächsten Ausschütthubs in die Vorschubrichtung der Produktdosis entspricht, die in der Ampulle 2 für eine letzte Ausschüttung noch verfügbar ist. Im Ausführungsbeispiel beträgt diese Restmenge acht Dosiseinheiten. Dementsprechend überdecken sich der Zeiger 32 und die Dosismarke 31, die der Dosis von acht Dosiseinheiten zugeordnet ist. Die ausschüttbare Restmenge wird durch einen Anschlag definiert, der zwischen der Kolbenstange 8 und der Antriebsvorrichtung 10/18 wirksam ist. Dieser Anschlag begrenzt die effektive Länge der Kolbenstange 8, d.h. die Länge, um die die Kolbenstange 8 aus ihrer Position vor einer ersten Ausschüttung insgesamt bis zur Entleerung der Ampulle 2 in die Vorschubrichtung bewegt werden kann. Im Ausführungsbeispiel bildet die Kolbenstange 8 in ihrem hinteren Teil diesen Anschlag für die Mitnehmer 11, d.h. die Mitnehmer 11 können diesem Anschlag nicht elastisch ausweichen.

### Bezugszeichenliste:

- 1: erster oder vorderer Gehäuseabschnitt
- 2: Reservoir, Ampulle
- 3: zweiter oder hinterer Gehäuseabschnitt
- 4: Sperreinrichtung
- 5: Ausschüttanschlag
- 6: Gewinde
- 7: erstes Rastelement, Axialnut
- 8: Kolbenstange
- 10: Antriebsglied
- 11: Mitnehmer
- 12: Ausschütt- und Dosieranschlag
- 13: Dosierglied, innerer Dosierkörper
- 14: Dosierglied, äußerer Dosierkörper
- 15: Dosieranschlag
- 16: Gewinde
- 17: zweites Rastelement, Eingriffsglied
- 18: Betätigungselement
- 19: Priminggegenanschlag, Eingriffsglied
- 20: Führungskurve
- 21: erster Führungsabschnitt
- 22: zweiter Führungsabschnitt
- 23: Priminganschlag
- 24: Verbindungsabschnitt
- 25: Rotationsanschlag
- 26: Rotationsanschlag
- 30: Dosisskala
- 31: Dosismarke
- 32: Zeiger, Markierungslinie
- 33: -
- 34: Sichtfenster
- 34a: Lupe
- K: Kolben
- L: Translationsachse, Rotationsachse, Längsachse
- N: Injektionsnadel
- P: Schutzkappe

## Patentansprüche

1. Verabreichungsgerät für die Verabreichung eines fluiden Produkts, umfassend:
a) ein Gehäuse (1, 3) mit einem Reservoir (2) für das Produkt,
b) eine Abtriebsvorrichtung (K, 8), die auf das in dem Reservoir (2) enthaltene Produkt wirkt, um das Produkt auszuschütten,
c) und eine auf die Abtriebsvorrichtung (K, 8) wirkende Antriebsvorrichtung (10/18), die aus einer Ausschüttposition in eine Antriebsrichtung bis gegen einen Ausschüttanschlag (5) einen Ausschütthub (Ah) ausführt, um eine zu verabreichende Produktdosis auszuschütten,
**dadurch gekennzeichnet, dass**
d) ein Priminganschlag (23) für die Antriebsvorrichtung (10/18) vorgesehen ist, der einen der Entlüftung dienenden Priminghub (Ph) der Antriebsvorrichtung (10/18) in die Antriebsrichtung begrenzt, wobei der Priminghub (Ph) axial kürzer als ein maximaler Ausschütthub (Ahmax) ist,
e) und dass die Antriebsvorrichtung (10/18) aus einer Primingposition in die Antriebsrichtung bis gegen den Priminganschlag (23) und quer zu der Antriebsrichtung bis in die Ausschüttposition bewegbar ist.

2. Verabreichungsgerät nach Anspruch 1, **dadurch gekennzeichnet, dass** ein die Ausschüttposition bestimmender Anschlag (26) vorgesehen ist, der die Bewegung der Antriebsvorrichtung (10/18) aus der Primingposition in die Ausschüttposition begrenzt.

3. Verabreichungsgerät nach einem der vorhergehenden Ansprüche, **dadurch gekennzeichnet, dass** die Antriebsvorrichtung (10/18) aus der Ausschüttposition in die Primingposition bewegbar ist.

4. Verabreichungsgerät nach dem vorhergehenden Anspruch, **dadurch gekennzeichnet, dass** ein die Primingposition bestimmender Anschlag (25) vorgesehen ist, der die Bewegung der Antriebsvorrichtung (10/18) in die Primingposition begrenzt.

5. Verabreichungsgerät nach einem der vorhergehenden Ansprüche, **dadurch gekennzeichnet, dass** die Bewegung der Antriebsvorrichtung (10/18) aus der Primingposition in die Ausschüttposition eine Rotationsbewegung um eine in die Antriebsrichtung weisende Rotationsachse (L) ist.

6. Verabreichungsgerät nach einem der vorhergehenden Ansprüche, **dadurch gekennzeichnet, dass** eine Führungskurve (20) und ein in die Führungskurve (20) eingreifendes Eingriffsglied (19) den Priminganschlag (23) und einen von der Antriebsvorrichtung (10/18) gebildeten Priminggegenanschlag (19) bilden.

7. Verabreichungsgerät nach dem vorhergehenden Anspruch, **dadurch gekennzeichnet, dass** das Verabreichungsgerät ein Dosierglied (13/14) umfasst, das einen Dosieranschlag (15) für die Antriebsvorrichtung (10/18) bildet und für eine in oder gegen die Antriebsrichtung gerichtete axiale Verstellung des Dosieranschlags (15) mit dem Gehäuse (1, 3) bewegbar gekoppelt ist, vorzugsweise mittels eines Drehgelenks (6, 16), und dass die Antriebsvorrichtung (10/18) und das Dosierglied (13/14) in einem den Priminghub (Ph) bestimmenden Eingriff sind, indem sie die Führungskurve (20) und den Priminggegenanschlag (19) bilden.

8. Verabreichungsgerät nach einem der zwei vorhergehenden Ansprüche, **dadurch gekennzeichnet, dass** die Führungskurve (20) und der Priminggegenanschlag (19) an einander zugewandten, in Antriebsrichtung sich erstreckenden Mantelflächen zweier Körper gebildet sind, von denen der eine mit der Antriebsvorrichtung (10/18) oder mit einem Bestandteil (18) der Antriebsvorrichtung (10/18) und der andere (13/14) mit dem Gehäuse (1, 3) gekoppelt ist.

9. Verabreichungsgerät nach Anspruch 6, **dadurch gekennzeichnet, dass** die Antriebsvorrichtung und das Gehäuse über die Führungskurve und den Priminggegenanschlag in Eingriff sind, um den Priminghub zu bestimmen.

10. Verabreichungsgerät nach einem der vier vorhergehenden Ansprüche, **dadurch gekennzeichnet, dass** die Führungskurve (20) einen ersten Führungsabschnitt (21) und einen zweiten Führungsabschnitt (22) umfasst und das Eingriffsglied (19) in den ersten Führungsabschnitt (21) eingreift, wenn die Antriebsvorrichtung (10/18) den Ausschütthub (Ah) ausführt, und in den zweiten Führungsabschnitt (22) eingreift, wenn die Antriebsvorrichtung (10/18) den Priminghub (Ph) ausführt.

11. Verabreichungsgerät nach dem vorhergehenden Anspruch, **dadurch gekennzeichnet, dass** das Eingriffsglied (19) nur in einen der Führungsabschnitte (21, 22) eingreifen und über einen Verbindungsabschnitt (24) der Führungskurve (20) aus dem einen der Führungsabschnitte (21, 22) in den anderen bewegt werden kann.

12. Verabreichungsgerät nach einem der vorhergehenden Ansprüche, **dadurch gekennzeichnet, dass** das Verabreichungsgerät ein Dosierglied (13/14) umfasst, das einen Dosieranschlag (15) bildet und relativ zu dem Gehäuse (1, 3) eine Dosierbewegung ausführt, durch die ein die Länge des Ausschütthubs (Ah) bestimmender, in Antriebsrichtung gemessener Abstand zwischen dem Ausschüttanschlag (5) und dem Dosieranschlag (15) verstellt wird.

13. Verabreichungsgerät nach dem vorhergehenden Anspruch, **dadurch gekennzeichnet, dass** die Antriebsvorrichtung (10/18) gegen die Antriebsrichtung bis gegen den Dosieranschlag (15) bewegbar ist.

14. Verabreichungsgerät nach einem der zwei vorhergehenden Ansprüche, **dadurch gekennzeichnet, dass** die Dosierbewegung des Dosierglieds (13/14) eine in oder gegen die Antriebsrichtung gerichtete Translationsbewegung umfasst oder ist.

15. Verabreichungsgerät nach einem der drei vorhergehenden Ansprüche, **dadurch gekennzeichnet, dass** die Dosierbewegung des Dosierglieds (13/14) eine Kombination aus einer in oder gegen die Antriebsrichtung gerichteten Translationsbewegung und einer Rotationsbewegung um eine in die Antriebsrichtung weisende Rotationsachse (L) ist.

16. Verabreichungsgerät nach einem der vier vorhergehenden Ansprüche, **dadurch gekennzeichnet, dass** das Dosierglied (13/14) zwei miteinander verbundene Dosierkörper umfasst, von denen der eine einen inneren Dosierkörper (13) und der andere einen äußeren Dosierkörper (14) bildet, zwischen denen ein Spalt verbleibt, in den das Gehäuse (1, 3) ragt.

17. Verabreichungsgerät nach dem vorhergehenden Anspruch, **dadurch gekennzeichnet, dass** einer (13) der Dosierkörper mit dem Gehäuse (1, 3) in einem Gewindeeingriff um eine in die Antriebsrichtung weisende Rotationsachse (L) ist und den anderen (14) der Dosierkörper bei einer Rotationsbewegung um die Rotationsachse (L) zumindest translatorisch mitnimmt.

18. Verabreichungsgerät nach einem der vorhergehenden Ansprüche, **dadurch gekennzeichnet, dass** die Antriebsvorrichtung (10/18) ein Antriebsglied (10) und ein Betätigungselement (18) umfasst, die in und gegen die Antriebsrichtung verschiebegesichert und um eine in die Antriebsrichtung weisende Rotationsachse (L) relativ zueinander verdrehbar miteinander verbunden sind, wobei das Antriebsglied (10) in Eingriff mit der Abtriebsvorrichtung (K, 8) ist, um die Abtriebsvorrichtung (K, 8) bei einer Bewegung der Antriebsvorrichtung (10/18) in die Antriebsrichtung mitzunehmen, und das Betätigungselement (18) einer Betätigung der Antriebsvorrichtung (10/18) dient.

19. Verabreichungsgerät nach dem vorhergehenden Anspruch, **dadurch gekennzeichnet, dass** das Betätigungselement (18) einen Priminggegenanschlag (19) für den Priminganschlag (23) bildet.

20. Verabreichungsgerät nach einem der vorhergehenden Ansprüche, **dadurch gekennzeichnet, dass** die Abtriebsvorrichtung (K, 8) einen Kolben (K), der in dem Reservoir (2) auf einen Auslass des Reservoirs (2) zu bewegbar ist, um Produkt auszuschütten, und eine Kolbenstange (8) aufweist, die mit der Antriebsvorrichtung (10/18) in Eingriff ist.

21. Verabreichungsgerät nach dem vorhergehenden Anspruch, **dadurch gekennzeichnet, dass** die Kolbenstange (8) wenigstens eine axial sich erstreckende Zahnreihe (9) und die Antriebsvorrichtung (10/18) wenigstens einen Mitnehmer (11) aufweist, der in die wenigstens eine Zahnreihe (9) eingreift, damit die Antriebsvorrichtung (10/18) die Abtriebsvorrichtung (K, 8) in die Antriebsrichtung mitnimmt.

22. Verabreichungsgerät nach einem der vorhergehenden Ansprüche, **dadurch gekennzeichnet, dass** das Verabreichungsgerät ein Injektionsgerät mit einer Injektionskanüle von höchstens 30 Gauge, vorzugsweise 31 Gauge oder 32 Gauge, oder eine Injektionskanüle mit einem Außendurchmesser von höchstens 320 µm und einem nicht in ISO 9626 spezifizierten Innendurchmesser ist, wobei die Wandstärke dünner als in ISO 9626 spezifiziert ist.

## Claims

1. An administration device for the administration of a fluid product including
a) a housing (1, 3) having a reservoir (2) for the product,
b) a driven device (K, 8) which acts on the product contained in the reservoir (2) to expel the product, and
c) a drive device (10/18) which acts on the driven device (K, 8) and which performs an expulsion stroke (Ah) from an expulsion position in a drive direction until encountering an expulsion abutment (5) in order to expel a product dose to be administered,
**characterised in that**
d) there is provided a priming abutment (23) for the drive device (10/18) which limits a priming stroke (Ph) of the drive device (10/18) serving for venting purposes in the drive direction, wherein the priming stroke (Ph) is axially shorter than a maximum expulsion stroke (Ahmax), and
e) the drive device (10/18) is movable from a priming position in the drive direction until encountering the priming abutment (23) and transversely with respect to the drive direction into the expulsion position.

2. An administration device according to claim 1 **characterised in that** there is provided an abutment (26) which determines the expulsion position and which limits the movement of the drive device (10/18) from the priming position into the expulsion position.

3. An administration device according to one of the preceding claims **characterised in that** the drive device (10/18) is movable out of the expulsion position into the priming position.

4. An administration device according to the preceding claim **characterised in that** there is provided an abutment (25) which determines the priming position and which limits the movement of the drive device (10/18) into the priming position.

5. An administration device according to one of the preceding claims **characterised in that** the movement of the drive device (10/18) out of the priming position into the expulsion position is a rotational movement about axis of rotation (L) pointing in the drive direction.

6. An administration device according to one of the preceding claims **characterised in that** a guide curve (20) and an engagement member (19) engaging into the guide curve (20) form the priming abutment (23) and a priming counter-abutment (19) which is formed by the drive device (10/18).

7. An administration device according to the preceding claim **characterised in that** the administration device includes a metering member (13/14) which forms a metering abutment (15) for the drive device (10/18) and for an axial displacement of the metering abutment (15) which is directed in or in opposite relationship to the drive direction is coupled movably to the housing (1, 3), preferably by means of a pivot (6, 16), and that the drive device (10/18) and the metering member (13/14) are in an engagement which determines the priming stroke (Ph) insofar as they form the guide curve (20) and the priming counter-abutment (19).

8. An administration device according to one of the two preceding claims **characterised in that** the guide curve (20) and the priming counter-abutment (19) are formed at mutually facing peripheral surfaces extending in the drive direction, of two bodies of which the one is coupled to the drive device (10/18) or to a component part (18) of the drive device (10/18) and the other (13/14) is coupled to the housing (1, 3).

9. An administration device according to claim 6 **characterised in that** the drive device and the housing are in engagement by way of the guide curve and the priming counter-abutment in order to determine the priming stroke.

10. An administration device according to one of the four preceding claims **characterised in that** the guide curve (20) includes a first guide portion (21) and a second guide portion (22) and the engagement member (19) engages into the first guide portion (21) when the drive device (10/18) performs the expulsion stroke (Ah) and it engages into the second guide portion (22) when the drive device (10/18) performs the priming stroke (Ph).

11. An administration device according to the preceding claim **characterised in that** the engagement member (19) can engage only into one of the guide portions (21, 22) and can be moved by way of a connecting portion (24) of the guide curve (20) out of one of the guide portions (21, 22) into the other.

12. An administration device according to one of the preceding claims **characterised in that** the administration device includes a metering member (13/14) which forms a metering abutment (15) and which performs relative to the housing (1, 3) a metering movement by which a spacing between the expulsion abutment (5) and the metering abutment (15), which determines the length of the expulsion stroke (Ah) and which is measured in the drive direction, is adjusted.

13. An administration device according to the preceding claim **characterized in that** the drive device (10/18) is movable in opposite relationship to the drive direction to a position against the metering abutment (15).

14. An administration device according to one of the two preceding claims **characterised in that** the metering movement of the metering member (13/14) includes or is a translatory movement directed in or in opposite relationship to the drive direction.

15. An administration device according to one of the three preceding claims **characterised in that** the metering movement of the metering member (13/14) is a combination of a translatory movement directed in or in opposite relationship to the drive direction and a rotational movement about an axis of rotation (L) pointing in the drive direction.

16. An administration device according to one of the four preceding claims **characterised in that** the metering member (13/14) includes two metering bodies which are connected together and of which the one forms an inner metering body (13) and the other forms an outer metering body (14) between which there remains a gap into which the housing (1, 3) projects.

17. An administration device according to the preceding claim **characterised in that** one (13) of the metering bodies is in threaded engagement with the housing (1, 3) about axis of rotation (L) pointing in the drive direction and entrains at least in a translatory movement the other one (14) of the metering bodies in a rotational movement about the axis of rotation (L).

18. An administration device according to one of the preceding claims **characterised in that** the drive device (10/18) includes a drive member (10) and an actuating element (18) which are secured against displacement in and in opposite relationship to the drive direction and which are connected together rotatably relative to each other about an axis of rotation (L) facing in the drive direction, wherein the drive member (10) is in engagement with the driven device (K, 8) in order to entrain the driven device (K, 8) in a movement of the drive device (10/18) in the drive direction and the actuating element (18) serves for actuation of the drive device (10/18).

19. An administration device according to the preceding claim **characterised in that** the actuating element (18) forms a priming counter-abutment (19) for the priming abutment (23).

20. An administration device according to one of the preceding claims **characterised in that** the driven device (K, 8) has a piston (K) which is movable in the reservoir (2) towards an outlet of the reservoir (2) to expel product and a piston rod (8) which is in engagement with the drive device (10/18).

21. An administration device according to the preceding claim **characterised in that** the piston rod (8) has at least one axially extending row of teeth (9) and the drive device (10/18) has at least one entrainment portion (11) which engages into the at least one row of teeth (9) so that the drive device (10/18) entrains the driven device (K, 8) in the drive direction.

22. An administration device according to one of the preceding claims **characterised in that** the administration device is an injection device having an injection cannula of at most 30 gauge, preferably 31 gauge or 32 gauge, or an injection cannula of an outside diameter of at most 320 µm and an inside diameter which is not specified in ISO 9626, wherein the wall thickness is thinner than is specified in ISO 9626.

## Revendications

1. Appareil d'administration pour l'administration d'un produit fluide, comprenant :
a) un logement (1, 3) avec un réservoir (2) pour le produit,
b) un dispositif d'expulsion (K, 8) qui agit sur le produit contenu dans le réservoir (2) pour distribuer le produit,
c) et, agissant sur le dispositif d'expulsion (K, 8), un dispositif de propulsion (10/18) qui exécute une course de distribution (Ah) depuis une position de distribution dans une direction de propulsion jusqu'à ce qu'il atteigne une butée de distribution (5), afin de distribuer une dose de produit à administrer,
**caractérisé en ce que**
d) une butée d'amorçage (23) pour le dispositif de propulsion (10/18) est prévue, laquelle, dans la direction de propulsion, limite une course d'amorçage (Ph) du dispositif de propulsion (10/18) servant à l'aspiration, la course d'amorçage (Ph) étant axialement plus courte qu'une course de distribution maximale (Ahmax),
e) et **en ce que** le dispositif de propulsion (10/18) peut être déplacé depuis une position d'amorçage dans la direction de propulsion jusqu'à ce qu'il atteigne la butée d'amorçage (23) et transversalement à la direction de propulsion jusque dans la position de distribution.

2. Appareil d'administration selon la revendication 1, **caractérisé en ce qu'**une butée (26) définissant la position de distribution est prévue, laquelle limite le mouvement du dispositif de propulsion (10/18) depuis la position d'amorçage dans la position de distribution.

3. Appareil d'administration selon l'une quelconque des revendications précédentes, **caractérisé en ce que** le dispositif de propulsion (10/18) peut être déplacé depuis la position de distribution dans la position d'amorçage.

4. Appareil d'administration selon la revendication précédente, **caractérisé en ce qu'**une butée (25) définissant la position d'amorçage est prévue, laquelle limite le mouvement du dispositif de propulsion (10/18) dans la position d'amorçage.

5. Appareil d'administration selon l'une quelconque des revendications précédentes, **caractérisé en ce que** le mouvement du dispositif de propulsion (10/18) depuis la position d'amorçage dans la position de distribution est un mouvement de rotation autour d'un axe de rotation (L) orienté dans la direction de propulsion.

6. Appareil d'administration selon l'une quelconque des revendications précédentes, **caractérisé en ce qu'**une courbe de guidage (20) et un élément d'engagement (19) venant en prise dans la courbe de guidage (20) constituent la butée d'amorçage (23) et une contre-butée d'amorçage (19) formée par le dispositif de propulsion (10/18).

7. Appareil d'administration selon la revendication précédente, **caractérisé en ce que** l'appareil d'administration comprend un élément de dosage (13/14) qui forme une butée de dosage (15) pour le dispositif de propulsion (10/18) et qui, de préférence au moyen d'un joint articulé (6, 16), est couplé de manière mobile avec le logement (1, 3) pour un réglage axial de la butée de dosage (15) dirigé vers ou contre la direction de propulsion, et **en ce que** le dispositif de propulsion (10/18) et l'élément de dosage (13/14) sont dans un engagement définissant la course d'amorçage (Ph), la courbe de guidage (20) et la contre-butée d'amorçage (19) étant formées par eux.

8. Appareil d'administration selon l'une quelconque des deux revendications précédentes, **caractérisé en ce que** la courbe de guidage (20) et la contre-butée d'amorçage (19) sont constituées de surfaces latérales des deux corps s'étendant dans la direction de propulsion et tournées l'une vers l'autre, parmi lesquelles la une est couplée avec le dispositif de propulsion (10/18) ou avec une partie (18) du dispositif de propulsion (10/18) et l'autre (13/14) avec le logement (1, 3).

9. Appareil d'administration selon la revendication 6, **caractérisé en ce que** le dispositif de propulsion et le logement sont en prise au-dessus de la courbe de guidage et de la contre-butée d'amorçage, afin de définir la course d'amorçage.

10. Appareil d'administration selon l'une quelconque des quatre revendications précédentes, **caractérisé en ce que** la courbe de guidage (20) comprend un premier tronçon de guidage (21) et un second tronçon de guidage (22) et l'élément d'engagement (19) vient en prise dans le premier tronçon de guidage (21), lorsque le dispositif de propulsion (10/18) exécute la course de distribution (Ah), et vient en prise dans le second tronçon de guidage (22), lorsque le dispositif de propulsion (10/18) exécute la course d'amorçage (Ph).

11. Appareil d'administration selon la revendication précédente, **caractérisé en ce que** l'élément d'engagement (19) peut uniquement venir en prise dans un des tronçons de guidage (21, 22) et être déplacé depuis le un des tronçons de guidage (21, 22) vers l'autre au travers d'un tronçon de raccordement (24) de la courbe de guidage (20).

12. Appareil d'administration selon l'une quelconque des revendications précédentes, **caractérisé en ce que** l'appareil d'administration comprend un élément de dosage (13/14) qui forme une butée de dosage (15) et qui exécute, par rapport au logement (1, 3), un mouvement de dosage, par lequel est réglée une distance entre la butée de distribution (5) et la butée de dosage (15), mesurée dans la direction de propulsion et définissant la longueur de la course de distribution (Ah).

13. Appareil d'administration selon la revendication précédente, **caractérisé en ce que** le dispositif de propulsion (10/18) peut être déplacé contre la direction de propulsion jusqu'à son arrivée contre la butée de dosage (15).

14. Appareil d'administration selon l'une quelconque des deux revendications précédentes, **caractérisé en ce que** le mouvement de dosage de l'élément de dosage (13/14) comprend ou est un mouvement de translation dirigé vers ou contre la direction de propulsion.

15. Appareil d'administration selon l'une quelconque des trois revendications précédentes, **caractérisé en ce que** le mouvement de dosage de l'élément de dosage (13/14) est une combinaison d'un mouvement de translation dirigé vers ou contre la direction de propulsion et d'un mouvement de rotation autour d'un axe de rotation (L) orienté dans la direction de propulsion.

16. Appareil d'administration selon l'une quelconque des quatre revendications précédentes, **caractérisé en ce que** l'élément de dosage (13/14) comprend deux corps de dosage reliés l'un à l'autre, parmi lesquels le un forme un corps de dosage interne (13) et l'autre un corps de dosage externe (14), entre lesquels il reste une fente dans laquelle le logement (1, 3) se dresse.

17. Appareil d'administration selon la revendication précédente, **caractérisé en ce qu'**un (13) des corps de dosage est dans un engagement fileté avec le logement (1,3) autour d'un axe de rotation (L) orienté dans la direction de propulsion et entraîne, au moins par translation, l'autre (14) des corps de dosage lors d'un mouvement de rotation autour de l'axe (L).

18. Appareil d'administration selon l'une quelconque des revendications précédentes, **caractérisé en ce que** le dispositif de propulsion (10/18) comprend un élément de propulsion (10) et un élément d'actionnement (18) qui sont bloqués vers et contre la direction de propulsion et qui sont reliés l'un à l'autre de manière articulée l'un par rapport à l'autre autour d'un axe de rotation (L) orienté dans la direction de propulsion, l'élément de propulsion (10) étant en prise avec le dispositif d'expulsion (K, 8), afin d'entraîner le dispositif d'expulsion (K, 8) par un déplacement du dispositif de propulsion (10/18) dans la direction de propulsion, et l'élément d'actionnement (18) servant à l'actionnement du dispositif de propulsion (10/18).

19. Appareil d'administration selon la revendication précédente, **caractérisé en ce que** l'élément d'actionnement (18) forme une contre-butée d'amorçage (19) pour la butée d'amorçage (23).

20. Appareil d'administration selon l'une quelconque des revendications précédentes, **caractérisé en ce que** le dispositif d'expulsion (K, 8) est pourvu d'un piston (K) qui, dans le réservoir (2), peut être déplacé vers une sortie du réservoir (2) afin de distribuer le produit, et d'une tige de piston (8) qui est en prise avec le dispositif de propulsion (10/18).

21. Appareil d'administration selon la revendication précédente, **caractérisé en ce que** la tige de piston (8) est pourvue au moins d'une rangée de dents (9) s'étendant axialement et le dispositif de propulsion (10/18) est pourvu d'au moins un doigt d'entraînement (11) qui vient en prise dans la au moins une rangée de dents (9), de sorte que le dispositif de propulsion (10/18) entraîne le dispositif d'expulsion (K, 8) dans la direction de propulsion.

22. Appareil d'administration selon l'une quelconque des revendications précédentes, **caractérisé en ce que** l'appareil d'administration est un appareil d'injection avec une aiguille d'injection d'au plus 30 Gauge, de préférence 31 Gauge ou 32 Gauge, ou une aiguille d'injection avec un diamètre externe d'au plus 320 µm et un diamètre interne non certifié selon ISO 9626, l'épaisseur de la paroi étant plus mince que celle certifiée selon ISO 9626.
